# EUROPEAN PATENT SPECIFICATION

(11) **EP 2 380 022 B1**
(45) Date of publication and mention of the grant of the patent: **05.04.2017**
(21) Application number: 09787377.2
(22) Date of filing: 04.12.2009
(51) Int. Cl.: G01N 33/543

(54) **SENSING DEVICE FOR DETECTING TARGET ELEMENTS IN A FLUID**
WAHRNEHMUNGSVORRICHTUNG ZUM NACHWEISEN VON ZIELELEMENTEN IN EINER FLÜSSIGKEIT
DISPOSITIF DE DÉTECTION PERMETTANT DE DÉTECTER DES ÉLÉMENTS CIBLES DANS UN FLUIDE

(30) Priority: 11.12.2008 EP 08171277
(43) Date of publication of application: 26.10.2011
(73) Proprietor: Koninklijke Philips N.V., 5656 AE Eindhoven (NL)
(72) Inventor: WIMBERGER-FRIEDL, Reinhold, NL-5656 AE Eindhoven (NL); DE WITZ, Christianne, R., M., NL-5656 AE Eindhoven (NL)
(74) Representative: Kroeze, Johannes Antonius
(86) International application number: PCT/IB2009/055496
(87) International publication number: WO 2010/067279

(56) References cited:
- HELMSTETTER C E: "Preferential release of one daughter cell during division of immobilized chinese hamster ovary cells." BIOTECHNOLOGY AND BIOENGINEERING 20 FEB 1995, vol. 45, no. 4, 20 February 1995 (1995-02-20), pages 374-378, XP002570737 ISSN: 0006-3592
- MENDOZA L G ET AL: "High-throughput microarray-based enzyme-linked immunosorbent assay (ELISA)." BIOTECHNIQUES OCT 1999, vol. 27, no. 4, October 1999 (1999-10), pages 778-780 , 782, XP002570738 ISSN: 0736-6205

## Description

### FIELD OF THE INVENTION

The present invention relates to a sensing device and a target element detection apparatus for detecting target elements in a fluid. The invention relates further to a method for manufacturing the sensing device.

### BACKGROUND OF THE INVENTION

US 2002/0028463 A1 discloses a sensing device for detecting target elements in a fluid, wherein the target elements are captured by capture elements located on a metal layer. Thus, also the captured target elements are located on the metal layer. This limits the group of detection methods that could be used for detecting the captured target elements, i.e. only a limited amount of different detection methods can be used for precisely detecting the captured target elements. For example, optical methods have to use radiation, which in the present case has to traverse the fluid before scanning the captured target elements. The radiation can be scattered and/or absorbed by the fluid, thereby decreasing the accuracy of the detection of the captured target elements.

Mendoza, L.G. et al. (BioTechniques 27(4), 1999, pages 778-788) describe a biochip for high throughput multiplexed enzyme-linked immunosorbent assays. The biochip consists of a glass plate containing 96 wells formed by an enclosing hydrophobic Teflon® mask. Each well contains four identical 36-elemnt arrays comprising 8 different antigens and a marker protein. Non-specific binding sites were blocked by pre-incubating with blocker casein.

Hehnstetter, C.E. (Biotechnology and Bioengineering 45, 1995, pages 374-378) describes CHO cells attached to tiny adhesion sites in polyHEMA coated glass. The adhesive sites were produced by placing a metal mask with 8 µm diameter holes on top of the coated glass and exposing the sandwich to glow discharge treatment to obtain said adhesive sites in said holes due to poly HEMA removal. After cell division only one cell remained attached to the adhesive site.

### SUMMARY OF THE INVENTION

It is an object of the present invention to provide a sensing device, a target element detection apparatus, and a manufacturing method for detecting target elements in a fluid.

In a first aspect of the present invention a sensing device being usable for detecting target elements in a fluid is presented, wherein the sensing device comprises:
- a substrate having first regions and second regions;
- a patterned first layer on the substrate covering the second regions such that the first regions are not covered by the first layer; and
- capture elements arranged in the first regions for capturing the target elements, wherein the capture elements have been applied by providing a solution containing the capture elements in the first regions and on the first layer.

A patterned first layer is provided on a substrate in order to provide first regions which are not covered by the patterned layer and second regions which are covered. The uncovered first regions are used as capture areas and capture elements are provided at the first regions. Since the first layer can be patterned very precisely by known techniques, the first regions and, thus, the regions comprising the capture elements can be defined very precisely. This allows for an improved accuracy in detecting the captured target elements. Furthermore, the first layer does generally not influence the set of detection methods that could be used for detecting the captured target elements, because the capture elements are not located on the first layer, and the material of the substrate can be chosen freely such that a desired detection method can be used for detecting the captured target elements. For example, the material of the substrate can be transparent to visible light allowing optically detecting the captured target elements without the need that radiation traverses the fluid before scanning the target elements. Thus, the sensing device allows using a larger number of different detection methods for precisely detecting the target elements.

The sensing device can be a device that comprises a detection unit for detecting the captured target elements, or the sensing device can be a device that interacts with a target element detection apparatus being adapted to detect the target elements captured in the sensing device. If the sensing device comprises a detection unit, it can further comprise a property determination unit for determining a property of the fluid based on the detected captured target elements, in particular, based on the amount of the detected captured target elements. If the target element detection apparatus detects the captured target elements, it can further comprise a property determination unit for determining a property of the fluid based on the detected captured target elements, in particular, based on the amount of the detected captured target elements.

The sensing device preferably further comprises a blocking second layer which is arranged on the first layer for reducing a binding of capture elements on the second regions. Such a provision of a second blocking layer is advantageous as capture elements will be bound in the second regions with a reduced probability or not at all, i.e. the capture elements will mainly or only be provided in the first regions.

The blocking layer preferably comprises a material which is hydrophilic.

Preferentially, the first regions are pretreated to improve their binding capacity for binding the capture elements. The first regions can be conditioned to enable a homogeneous and/or dense distribution of capture elements.

It is preferred that the substrate is at least at the first regions transparent. It is further preferred that the complete substrate is transparent. A transparent substrate is advantageous as it allows an optical detection of captured target elements in the sensing device.

The first layer is a metal layer. The metal layer is preferentially patterned by means of soft lithography. The use of a metal as a first layer is advantageous as particularly a metal layer can be patterned very precisely. Accordingly, the accuracy of the capture areas and, thus, of the detection of the captured target elements can be further improved.

It is further preferred that the capture elements are applied by providing a solution containing the capture elements onto the substrate and the first layer. It is further preferred that the capture elements are applied by printing spots of a solution containing the capture elements. A single printed spot can cover multiple first regions. Since the first regions, i.e. the capture regions, are defined by the patterned first layer and not by the precision of the provision of the solution containing the capture elements, the requirements with respect to the accuracy of the provision of the solution containing the target elements can be reduced, without reducing the accuracy of the definition of the capture regions. This allows to use less sophisticated methods for providing the solution, for example, inkjet printing.

It is further preferred that the first regions have a rectangular shape. This allows adapting the shape of the first regions to a detection of the target elements by using a CCD camera.

The sensing device is preferentially adapted to be cooperable with a target element detection apparatus for allowing the target element detection apparatus to detect the target elements in the fluid, if they are captured by the capture elements arranged at the first regions. The sensing device is, for example, a sensing cartridge being adapted for being insertable into the target element detection apparatus. This allows using disposable sensing devices, which can be used with the same target element detection apparatus.

In a further aspect of the present invention a target element detection apparatus for detecting target elements in a fluid is presented, wherein the target element detection apparatus is adapted to be cooperable with a sensing device, wherein the sensing device comprises:
- a substrate having first regions and second regions,
- a patterned first layer on the substrate covering the second regions such that the first regions are not covered by the first layer, and
- capture elements arranged in the first regions for capturing the target elements, wherein the capture elements have been applied by providing a solution containing the capture elements in the first regions and on the first layer,
wherein the target element detection apparatus is adapted to detect the target elements in the fluid, if they are captured by the capture elements arranged in the first regions.

In a further aspect of the invention a method for manufacturing a sensing device being usable for detecting target elements in a fluid is presented, wherein the method comprises the steps of:
- providing a substrate,
- providing a first layer on a substrate,
- patterning the first layer obtaining first regions and second regions, wherein the second regions are covered by the patterned first layer and the first regions are not covered by the patterned first layer, and
- providing capture elements for capturing the target elements of the fluid in the first regions, wherein the capture elements are provided by providing a solution containing the capture elements in the first regions and on the first layer.

It shall be understood that the sensing device according to claim 1, the target element detection apparatus according to claim 10 and the method for manufacturing a sensing device according to claim 11 have similar and/or identical preferred embodiments as defined in the dependent claims.

It shall be understood that a preferred embodiment of the invention can also be any combination of the dependent claims with the respective independent claims.

### BRIEF DESCRIPTION OF THE DRAWINGS

These and other aspects of the invention will be apparent from and elucidated with reference to the embodiments described hereinafter. In the following drawings:
Fig. 1 shows schematically and exemplarily an embodiment of a sensing device,
Fig. 2 shows schematically and exemplarily a sectional view of a detection surface,
Fig. 3 shows schematically and exemplarily a top view on a further detection surface,
Fig. 4 shows schematically and exemplarily a sectional view of an embodiment of a sensing cartridge,
Fig. 5 shows schematically and exemplarily an embodiment of a target element detection apparatus,
Fig. 6 shows schematically and exemplarily magnetic particles attached to a detection surface,
Fig. 7 shows a flow chart illustrating a method for manufacturing a s ensing device, and
Fig. 8 shows schematically and exemplarily a sectional view of a detection surface during manufacturing the sensing device according to the invention.

### DETAILED DESCRIPTION OF EMBODIMENTS

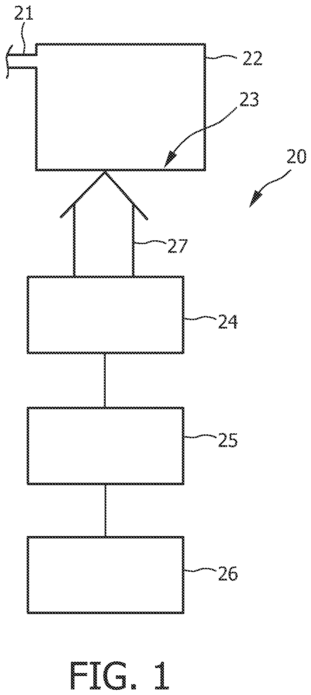

Fig. 1 shows schematically and exemplarily a sensing device 20 being usable for detecting target elements in a fluid. The sensing device 20 comprises a fluid inlet 21 for allowing a fluid to flow into the detection chamber 22. The fluid comprises target elements, which can be captured on a detection surface 23 of the detection chamber 22. The sensing device 20 further comprises a detection unit 24 being adapted to detect the captured target elements on the detection surface 23 by using a probe beam 27. The sensing device 20 further comprises a property determination unit 25 for determining a property of the fluid based on the detected captured target elements. The property that is determined by the property determination unit 25 is, for example, just the amount of target elements captured on the detection surface 23. The property of the fluid can also be that the amount of captured target elements exceeds a predefined threshold.

The sensing device 20 further comprises an output unit 26 for outputting a signal indicating the determined property of the fluid.

In an embodiment, the sensing device 20 does not comprise the output unit 26 and the property determination unit 25. In a further embodiment, the sensing device 20 does not even comprise the detection unit 24. The detection of the captured target elements may be performed by a separate detection device.

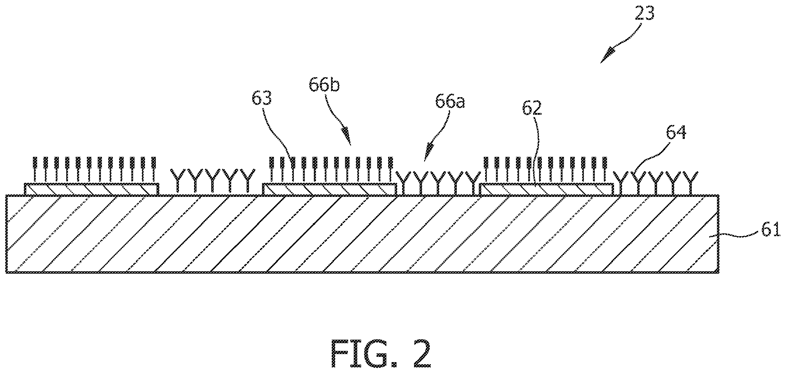

Fig. 2 shows schematically and exemplarity a cross section of the detection surface 23. The detection surface 23 comprises a substrate 61, a patterned first layer 62 and a blocking second layer 63 on the patterned first layer 62. Those areas (first regions 66a) of the substrate 61, which are not covered by the first layer 62, comprise capture elements 64.

For example, by means of soft lithography the first layer 62 can be patterned in a very precise way. Furthermore, soft lithography can enable a good definition of the shape of the first regions. On the first layer 62, which is implemented as a patterned metal layer, hydrophilic material can be applied before the capture elements are provided for forming the blocking second layer 63. It should be noted that substantially the capture elements can only bind to the regions, which are not covered by the metal layer and which are therefore not covered by the hydrophilic material. Accordingly, the accuracy of the shape of the spot areas can be high.

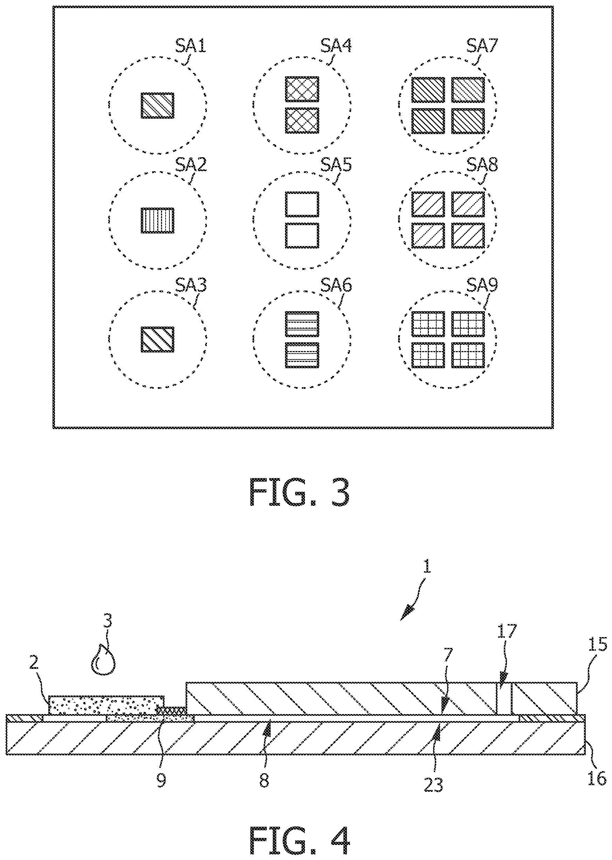

Fig. 3 shows schematically and exemplarily a top view of a detection surface according to a further embodiment. Here, spot areas SA1-SA9 are depicted. The dotted circles around the spot areas SA1-SA9 relate to an area which can be covered by capture spots containing capture elements after inkjet printing. For example, as seen with respect to the spot area SA9, several first regions, in this example four first regions, can be covered at the same time by one spot area.

The detection chamber 22 schematically and exemplarily shown in Fig. 1 can be a part of a sensing cartridge 1, which is schematically and exemplarily shown in Fig. 4. The sensing cartridge 1 comprises an optional filter element 2 for receiving the fluid 3 and for filtering the fluid. The sensing cartridge 1 comprises a first part 15 and a second part 16 and a capillary structure comprising a capillary fluidic channel 8 between the first part 15 and the second part 16. The fluid is guided to a detection location 7, where the detection surface 23 is provided, by capillary forces. The second part 16 corresponds to the substrate 61, on which the capture elements 64 are provided. In this embodiment, the sensing cartridge 1 comprises a further filter element 9 for filtering the fluid 3 while travelling through the fluidic channel 8. In other embodiments, the filter element 9 can be omitted. The sensing cartridge 1 further comprises a venting hole 17 for allowing air within the fluidic channel and/or fluid, which has passed the detection location 7, leaving the fluidic channel 8.

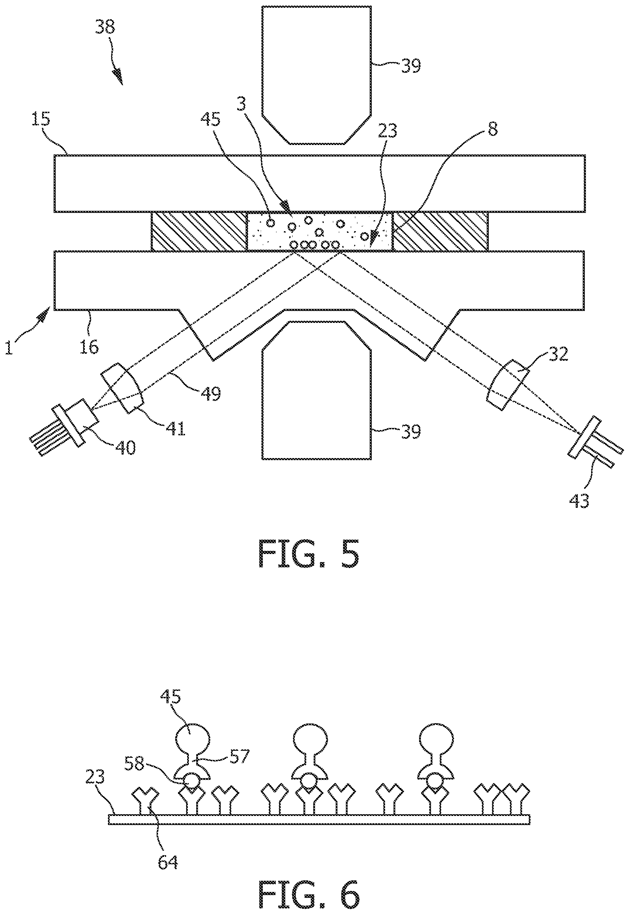

Fig. 5 shows schematically and exemplarily a cross section of the sensing cartridge 1 shown in Fig. 4 inserted into a target element detection apparatus 38. The target element detection apparatus 38 is adapted for detecting target elements in a fluid, if they are captured by the capture elements 64 arranged at the first regions 66a on the detection surface 23 of the sensing cartridge 1.

The target element detection apparatus 38 comprises, in this embodiment, a magnetic element 39, which provides a magnetic field for forcing magnetic particles 45 within the fluidic channel 8 onto the detection surface 23 of the second part 16 of the sensing cartridge 1. The magnetic particles 45 on the detection surface 23 are detected by, in this embodiment, illuminating this surface with a light beam 49 generated by a light source 40, which is, for example, a laser device or a LED, and by detecting the light reflected from the surface by a detector 43. The detector 43 is, for example, a photo detector like a photodiode or a two-dimensional camera like a CCD camera. Optical elements 41 and 32 can be arranged in the light beam 49 for generating parallel light or focusing the light beam 49, respectively. The optical elements 41, 32 are preferentially lenses.

The configuration sketched in Fig. 5 shows a detection of changes at a surface using the FTIR method (frustrated total internal reflection). If a beam of light reflects on the interface between a medium with a higher refractive index, for example the second part 16, and a lower refractive index, for example the fluid, there is a certain critical angle of incidence above which there is a situation of total internal reflection (TIR). The present detection configuration (regarding refractive indices and angle of incidence) is such that there is total internal reflection of the incoming beam. Although the light is totally reflected in such a situation, there is still penetration of the light in a very thin layer of the medium with the low refractive index. This is called evanescent light, the intensity of which decays exponentially in the low refractive index medium with a characteristic penetration depth of the order of the wavelength of the light. So, in practice the penetration depth is preferentially less than 0.5 micrometer. If magnetic particles stick to the surface, the optical properties of this very thin first fluid layer of preferentially about 0.5 micrometer are changed leading to a reduction of the reflected light beam. This is caused by absorption and scattering of the evanescent light. As a result the signal of the photo detector changes.

The detection of the target elements can, for example, be performed by a so-called sandwich assay, which will in the following be exemplarily described with reference to Fig. 6. Magnetic beads 45 are coated with a specific antibody 57 that attaches to a target element 58 present in the fluid. When the magnetic beads that are freely present in the fluid have reacted with the available target elements the beads are attracted to the detection surface 23 that has been coated with capture elements 64 being, for example, another antibody that can couple to a target element. After a sufficiently long reaction time the magnetic field is switched such that the magnetic beads are pulled upwards so that only the specifically bound beads with the correct target elements stay attached to the surface. At that moment the optical detector can be read out and gives a signal that carries the information on the amount of target elements on the detection surface. So the detection location, i.e. the first region of the detection surface 23, can be covered with a bio layer with antibodies.

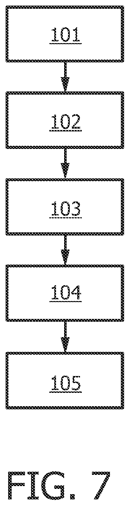

Fig. 7 shows a flow chart illustrating a method for manufacturing a sensing device being usable for detecting target elements in a fluid. In step 101, a substrate 61 is provided. In step 102, a first layer 62 is provided on the substrate 61. In step 103, the first layer 62 is patterned thus obtaining first and second regions 66a, 66b. The second regions 66b are covered by the patterned first layer 62 and the first regions 66a are not covered by the patterned first layer 62. In step 104, a blocking second layer 63 is provided on the first layer 62 and serves to reduce a binding of capture elements in the second regions 66b. In step 105, capture elements for capturing target elements in the fluid are provided in the first regions 66a.

In step 103, the first layer can be patterned by soft lithography in a very precise way. Furthermore, soft lithography can enable a good definition of the shape of the first regions, i.e. the capture regions. On the first layer 62, which is implemented as a patterned metal layer, hydrophilic material can be applied in step 104 for forming the blocking layer 63. It should be noted that the capture elements can mainly or only bind to the regions, which are not covered by the first layer and which are therefore not covered by the hydrophilic material.

The substrates according to the above embodiments can be at least partly transparent and can be used for a detection of target elements in biological, environmental or chemical samples. Preferably, the substrate is a part of a sensing device like a sensing cartridge. On or at the substrate a structured cover can be provided which may create fluidic channels. The sensing device may contain a sample port as well as waste chambers. Any reagents can be applied in wet or dry state on the substrates or in the fluidic channels to enable a bio- and/or bio-chemical analysis in a closed system. Optionally, film heaters can be integrated into the substrates as well as electrodes.

The sensing device and/or the target element detection apparatus described above can be used in the field of molecular diagnostics and immuno-sensing. Here, assays can be performed on parts of the area of a detection surface of the sensing device. The sensing device according to the invention can be combined with printed circuits and arrays of different capture probes like DNA arrays. The sensing device according to the invention can be advantageous as the sensitivity of an analysis can be improved, in particular, if weak signals are obtained.

The capture elements can be linked to the substrate by physisorption or a chemical reaction.

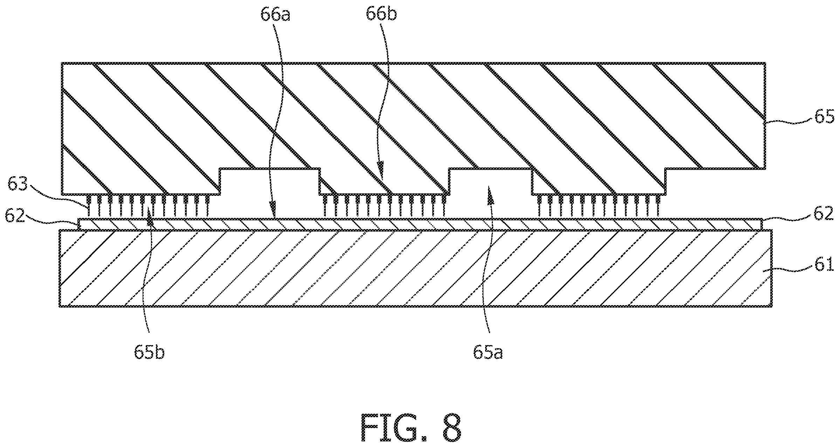

Fig. 8 shows schematically and exemplarily a sectional view of a detection surface during fabrication step 104, i.e. during the step of providing a blocking second layer 63. The detection surface comprises the substrate 61 and the first layer 62 on the substrate 61. As already mentioned above, the substrate 61 is preferentially a transparent carrier, and the first layer 62 is a layer of metal like gold, aluminum, platinum, palladium et cetera. The first layer 62 can be deposited on the substrate 61, for example, by means of evaporation. However, it should be noted that also other deposition methods can be applied. The thickness of the first layer 62 is, for example, several nanometers.

In Fig. 8, the detection surface is depicted during a micro-contact printing MCP step. A stamp 65 of PDMS, which has a structured or patterned surface, is impregnated with the second blocking layer 63 of, for example, a thiol. The stamp 65 can be dried before being applied to the first layer 62. The patterned surface of the stamp 65 can preferably refer to a negative image of the desired pattern of the first layer 62. In other words, the surface of the stamp 65 can comprise recess areas 65a and elevated areas 65b. The recessed areas 65a correspond to the first regions 66a and the elevated areas 65b correspond to the second regions 66b. When the stamp 65 has been applied to the surface of the first layer 62 and has been removed, a self-assembled monolayer SAM of the thiol is formed. Typically the thiol will form a chemical bond with the material of the first layer 62. Afterwards, the surface of the first layer 62 can be etched preferably by being immersed in an etching bath. In the etching bath, those areas (first regions 66a) which are not covered by the thiol monolayer, can be etched away to uncover the surface of the substrate 61. In other words, in those areas (second regions 66b), where the second blocking layer 63 of thiol is present, the first layer 62 is not etched away. On the other hand, in those areas in between (first regions 66a), i.e. without the second blocking layer 63, the first layer 62 is etched away.

The thiols can contain linear alkane chains of several carbon atoms length in order to have a good etching resistance. However, these thiols can be hydrophobic and are not preferred for use in the present invention. On the other hand, diblock thiols contain an alkane chain next to the thiol group but also a polyethyleneglycol (PEG) block opposite to the thiol group. Accordingly, a self-assembled monolayer with a polyethyleneglycol surface characteristic can be achieved. Such a self-assembled monolayer is sufficient for generating the patterned first layer, even if it only comprises a reduced etch resistance. This will, however, not hinder its application according to the invention as merely a thin layer must be etched.

In the first regions 66a capture elements can be linked to the substrate, for example, by physisorption. The physisorption can be used to bind antibodies on polymer surfaces. The capture elements can also be linked to the substrate by a chemical reaction to a functionalized surface like an epoxide or amine group. These can be created at the surface by a reaction with chloro- or alkoxy-silane with a terminal epoxy or amine group.

However, also other surface treatments may be applied to improve the density and functionality of the capture elements. The deposition and patterning of the first layer 62 can be performed before any surface treatment surface is performed. Thereafter, the capture elements can be provided in the first regions, for example, by means of a printing method, e.g. by inkjet printing. Here, a solution with the capture elements can be applied to the first regions. It should be noted that the size and pattern of the regions covered by the capture elements can be determined by the pattern of the first layer 62. Any capture elements being applied outside the first regions will be washed away as they can not adhere to the surface of the first layer. Preferably, a printed spot may cover several first regions which are defined by the patterned first layer. Accordingly, a sub-spot pixilation is possible. In addition, the density of the capture elements can be more uniform, as edges being defined by the drying rim of the printed spots will be eliminated. Moreover, the layout of the first regions can be adjusted, for example, to embody a rectangular characteristic.

The invention relates to the idea to provide a pattern of capture elements with a high definition having small features and/or precise or sharp boundaries. Small features are helpful with respect to an improved accessibility of the target elements to be captured. In particular providing a patterned metal layer can improve the high resolution of the sensor without having to apply the capture elements based on a high resolution applying method. In other words, the capture elements can be applied by means of inkjet printing.

The provision of the self-assembled monolayers (SAM) on the first layer is advantageous as a micro-contact printing may be used to apply the SAM. In addition, the SAM on the first layer can be used as an etch resisting layer during the etching of the first layer. Preferably, the SAM is hydrophilic.

In the above embodiments, the first layer 62 is a layer of metal like gold, aluminum, palladium, platinum et cetera. Furthermore, the thiols may be replaced by other functional groups which are reactive to the material of the chosen layer. Moreover, the PDMS can be replaced by another soft polymer material like hydrogels. The inkjet printing can be replaced by incubation, dip pen printing et cetera. The micro-contact printing can be replaced by capillary molding, screen printing, or lithographic techniques. The substrate can be glass or an injection molded or extruded polymer, like PMMA, PS, PC, COP, PET.

Although in the above described embodiments, the detection surface, which is schematically and exemplarily shown in Fig. 2, is integrated in certain sensing devices, in other embodiments, this detection surface can be integrated in another sensing device. The invention relates to any sensing device being usable for detecting target elements in a fluid, which comprises a substrate having first regions and second regions, a patterned first layer on the substrate covering the second regions such that the first regions are not covered by the first layer, and capture elements arranged in the first regions for capturing the target elements. In an embodiment, the sensing device can just be a detection surface formed by a substrate having first regions and second regions, a patterned first layer on the substrate covering the second regions such that the first regions are not covered by the first layer and capture elements arranged in the first regions for capturing the target elements.

A method for manufacturing a sensing device being useable for detecting target elements in a fluid in accordance with the invention comprises at least the steps of providing a substrate, providing a first layer on the substrate, patterning the first layer obtaining first and second regions, wherein the second regions are covered by the patterned first layer and the first regions are not covered by the patterned first layer, and providing capture elements for capturing the target elements of the fluid in the first regions. In an embodiment, the provision of the first layer on the substrate and the patterning of the first layer for obtaining first regions and second regions can be performed in a single step, wherein a patterned first layer is provided on the substrate. If the sensing device comprises more components, the method for manufacturing a sensing device being usable for detecting target elements in a fluid can comprise further steps, for example, integrating the detection surface in a sensing cartridge or in another more complex sensing device.

Sensing devices like sensing cartridges for biosensors or chemical-sensors are used in the field of diagnostics and can serve to detect low concentrations of analytes in samples. The analytes can be regarded as target elements that can be captured on a substrate with capture elements, which are, for example, antibodies for protein detection, single strands of DNA/RNA for DNA/RNA detection or molecules for drug detection. Moreover, biosensors with a multiplex system are known, where different species can be detected simultaneously. A method of providing different capture molecules on well defined positions is a printing method. If the target molecules are bound to capture elements any physical change resulting from such a bound can be detected.

The sensing device can comprise a bio-molecule array for a simultaneous monitoring of a plurality of interactions between biological molecules.

The patterned first layer can be compatible with many detection principles, in particular, optical detection, which, for example, uses fluorescence.

As already mentioned above, the second layer is preferentially a layer of polyethyleneglycol or of another hydrophilic material. The capture elements are preferentially antibodies or oligonucleotides.

The patterned first layer can be adapted to block light coming from the detection surface or the region above the detection surface.

The sensing device can be adapted to allow any suitable detection of the presence of particles, in particular, magnetic particles, on or near to a detection surface, based on any property of the particles, e.g. it can detect via magnetic methods (e.g. magnetoresistive, Hall, coils), optical methods (e.g. imaging, fluorescence, chemiluminescence, absorption, scattering, evanescent field techniques, surface plasmon resonance, Raman, etc.), sonic detection (e.g. surface acoustic wave, bulk acoustic wave, cantilever, quartz crystal etc), electrical detection (e.g. conduction, impedance, amperometric, redox cycling), combinations thereof, et cetera.

In an embodiment, the target elements are molecular targets. Molecular targets often determine the concentration and/or presence of larger moieties, e.g. cells, viruses, or fractions of cells or viruses, tissue extract, et cetera.

The target elements can be further processed prior to detection. An example of further processing is that materials are added or that the (bio)chemical or physical properties of the target elements are modified to facilitate detection.

The devices, apparatus and methods in accordance with the invention can be used with several biochemical assay types, e.g. binding/unbinding assay, sandwich assay, competition assay, displacement assay, enzymatic assay, et cetera.

The device, methods and apparatus in accordance with the invention can be suited for sensor multiplexing (i.e. the parallel use of different sensors and sensor surfaces), label multiplexing (i.e. the parallel use of different types of labels) and chamber multiplexing (i.e. the parallel use of different reaction chambers).

The device, methods and apparatus described in the present invention can be used as rapid, robust, and easy to use point-of-care biosensors for small sample volumes. The reaction chamber, i.e. the sensing cartridge, can be a disposable item to be used with a compact reader, i.e. the target element detection apparatus, containing one or more magnetic field generating means and one or more detection means. Also, the device, methods and apparatus of the present invention can be used in automated high-throughput testing. In this case, the detection chamber is, for example, a well plate or cuvette, fitting into an automated instrument.

The above mentioned magnet beads or magnetic particles are preferentially nano-particles having at least one dimension ranging between 3 nm and 5000 nm, preferably between 10 nm and 3000 nm, more preferred between 50 nm and 1000 nm.

Other variations to the disclosed embodiment can be understood and effected by those skilled in the art in practising the claimed invention, from a study of the drawings, the disclosure and the appended claims.

In the claims, the word "comprising" does not exclude other elements or steps, and the indefinite article "a" or "an" does not exclude a plurality.

A single unit or device may fulfill the functions of several items recited in the claims. The mere fact that certain measures are recited in mutually different dependent claims does not indicate that a combination of these measures cannot be used to advantage.

Any reference signs in the claims should not be construed as limiting the scope.

## Claims

1. Sensing device being usable for detecting target elements in a fluid, comprising:
- a substrate (61) having first regions (66a) and second regions (66b);
- a patterned first layer (62) on the substrate (61) covering the second regions (66b) such that the first regions (66a) are not covered by the first layer (62); and
- capture elements (64) arranged in the first regions (66a) for capturing the target elements,
wherein the first layer (62) is a metal layer.

2. Sensing device according to claim 1, further comprising:
a blocking second layer (63) arranged on the first layer (62) for reducing
a binding of capture elements in the second regions (66b).

3. Sensing device according to claim 2, wherein the blocking layer (63) comprises a material which is hydrophilic.

4. Sensing device according to claim 1, wherein the first regions (66a) are pretreated for improving their binding capacity for binding the capture elements.

5. Sensing device according to claim 1, wherein the substrate (61) is at least at the first regions (66a) transparent.

6. Sensing device according to claim 1, wherein the capture elements are applied by providing a solution containing the capture elements (64) onto the substrate (61) and the first layer (62).

7. Sensing device according to claim 1, wherein the capture elements (64) are applied by printing spots of a solution containing the capture elements (64), wherein a single printed spot covers multiple first regions (66a).

8. Sensing device according to claim 1, wherein the first regions (66a) are of a rectangular shape.

9. Sensing device according to claim 1, wherein the sensing device (1) is adapted to be cooperable with a target element detection apparatus (38) for allowing the target element detection apparatus (38) to detect the target elements in the fluid, if they are captured by the capture elements (64) arranged at the first regions (66a).

10. A target element detection apparatus for detecting target elements in a fluid (3), wherein the target element detection apparatus (38) is adapted to be cooperable with a sensing device (1), wherein the sensing device (1) comprises:
- a substrate (61) having first regions (66a) and second regions (66b),
- a patterned first layer (62) on the substrate (61) covering the second regions (66b) such that the first regions (66a) are not covered by the first layer (62), wherein the first layer (62) is a metal layer, and
- capture elements (64) arranged in the first regions (66a) for capturing the target elements,
wherein the target element detection apparatus (38) is adapted to detect the target elements in the fluid, if they are captured by the capture elements (64) arranged in the first regions (66a).

11. Method for manufacturing a sensing device being usable for detecting target elements in a fluid, comprising the steps of:
- providing a substrate (61);
- providing a first layer (62) on the substrate (61), wherein the first layer (62) is a metal layer;
- patterning the first layer (62) obtaining first regions (66a) and second regions (66b), wherein the second regions (66b) are covered by the patterned first layer (62) and the first regions (6a) are not covered by the patterned first layer (62); and
- providing capture elements (64) for capturing the target elements of the fluid in the first regions (66a).

## Patentansprüche

1. Erfassungsvorrichtung, die verwendbar ist, um Zielelemente in einer Flüssigkeit zu erfassen, die Folgendes umfasst:
- ein Substrat (61), das erste Bereiche (66a) und zweite Bereiche (66b) hat, und
- eine gemusterte erste Schicht (62) auf dem Substrat (61), die die zweiten Bereiche (66b) derart abdeckt, dass die ersten Bereiche (66a) durch die erste Schicht (62) nicht abgedeckt sind, und
- Erfassungselemente (64), die in den ersten Bereichen (66a) eingerichtet sind, um die Zielelemente zu erfassen,
wobei die erste Schicht (62) eine Metallschicht ist.

2. Erfassungsvorrichtung nach Anspruch 1, die ferner Folgendes umfasst:
eine zweite Blockierungsschicht (63), die auf der ersten Schicht (62) eingerichtet ist, um ein Binden von Erfassungselementen in den zweiten Bereichen (66b) zu verringern.

3. Erfassungsvorrichtung nach Anspruch 2, wobei die Blockierungsschicht (63) ein Material, das hydrophil ist, umfasst.

4. Erfassungsvorrichtung nach Anspruch 1, wobei die ersten Bereiche (66a) vorbehandelt sind, um ihre Bindungskapazität zum Binden der Erfassungselemente zu verbessern.

5. Erfassungsvorrichtung nach Anspruch 1, wobei das Substrat (61) mindestens an den ersten Bereichen (66a) durchsichtig ist.

6. Erfassungsvorrichtung nach Anspruch 1, wobei die Erfassungselemente durch Bereitstellen einer Lösung, die die Erfassungselemente (64) enthält, auf dem Substrat (61) und der ersten Schicht (62) aufgebracht werden.

7. Erfassungsvorrichtung nach Anspruch 1, wobei die Erfassungselemente (64) durch Drucken von Punkten einer Lösung, die die Erfassungselemente (64) enthält, aufgebracht werden, wobei ein einzelner gedruckter Punkt mehrere erste Bereiche (66a) abdeckt.

8. Erfassungsvorrichtung nach Anspruch 1, wobei die ersten Bereiche (66a) eine rechteckige Form haben.

9. Erfassungsvorrichtung nach Anspruch 1, wobei die Erfassungsvorrichtung (1) angepasst ist, um mit einem Zielelement-Erfassungsgerät (38) zusammenzuwirken zu können, um es dem Zielelement-Erfassungsgerät (38) zu erlauben, die Zielelemente in der Flüssigkeit zu erfassen, falls sie von den Erfassungselementen (64), die an den ersten Bereichen (66a) eingerichtet sind, erfasst werden.

10. Zielelement-Erfassungsgerät zum Erfassen von Zielelementen in einer Flüssigkeit (3), wobei das Zielelement-Erfassungsgerät (38) angepasst ist, um mit einer Erfassungsvorrichtung (1) zusammenwirken zu können, wobei die Erfassungsvorrichtung (1) Folgendes umfasst:
- ein Substrat (61), das erste Bereiche (66a) und zweite Bereiche (66b) hat,
- eine gemusterte erste Schicht (62) auf dem Substrat (61), die die zweiten Bereiche (66b) derart abdeckt, dass die ersten Bereiche (66a) durch die erste Schicht (62) nicht abgedeckt sind, wobei die erste Schicht (62) eine Metallschicht ist, und
- Erfassungselemente (64), die in den ersten Bereichen (66a) eingerichtet sind, um die Zielelemente zu erfassen,
wobei das Zielelement-Erfassungsgerät (38) angepasst ist, um die Zielelemente in der Flüssigkeit zu erfassen, falls sie von den Erfassungselementen (64), die in den ersten Bereichen (66a) eingerichtet sind, erfasst werden.

11. Verfahren zum Herstellen einer Erfassungsvorrichtung, die zum Erfassen von Zielelementen in einer Flüssigkeit verwendbar ist, das die folgenden Schritte umfasst:
- Bereitstellen eines Substrats (61),
- Bereitstellen einer ersten Schicht (62) auf dem Substrat (61), wobei die erste Schicht (62) eine Metallschicht ist,
- Mustern der ersten Schicht (62), wobei erste Bereiche (66a) und zweite Bereiche (66b) erhalten werden, wobei die zweiten Bereiche (66b) durch die gemusterte erste Schicht (62) abgedeckt sind, und die ersten Bereiche (66a) nicht durch die gemusterte erste Schicht (62) abgedeckt sind, und
- Bereitstellen von Erfassungselementen (64) zum Erfassen der Zielelemente der Flüssigkeit in den ersten Bereichen (66a).

## Revendications

1. Dispositif capteur utilisable pour détecter des éléments cibles dans un fluide, comprenant :
- un substrat (61) ayant des premières régions (66a) et des secondes régions (66b) ;
- une première couche à motifs (62) sur le substrat (61) couvrant les secondes régions (66b) de sorte que les premières régions (66a) ne soient pas couvertes par la première couche (62) ; et
- des éléments de capture (64) agencés dans les premières régions (66a) pour capturer les éléments cibles,
dans lequel la première couche (62) est une couche de métal.

2. Dispositif capteur selon la revendication 1, comprenant en outre :
une seconde couche de blocage (63) agencée sur la première couche (62) pour réduire une liaison d'éléments de capture dans les secondes régions (66b).

3. Dispositif capteur selon la revendication 2, dans lequel la couche de blocage (63) comprend un matériau qui est hydrophile.

4. Dispositif capteur selon la revendication 1, dans lequel les premières régions (66a) sont prétraitées pour améliorer leur capacité de liaison pour lier les éléments de capture.

5. Dispositif capteur selon la revendication 1, dans lequel le substrat (61) est transparent au moins au niveau des premières régions (66a).

6. Dispositif capteur selon la revendication 1, dans lequel les éléments de capture sont appliqués en fournissant une solution contenant les éléments de capture (64) sur le substrat (61) et la première couche (62).

7. Dispositif capteur selon la revendication 1, dans lequel les éléments de capture (64) sont appliqués en imprimant des points d'une solution contenant les éléments de capture (64), dans lequel un seul point imprimé couvre de multiples premières régions (66a).

8. Dispositif capteur selon la revendication 1, dans lequel les premières régions (66a) ont une forme rectangulaire.

9. Dispositif capteur selon la revendication 1, dans lequel le dispositif capteur (1) est adapté pour pouvoir coopérer avec un appareil de détection d'élément cible (38) pour permettre à l'appareil de détection d'élément cible (38) de détecter les éléments cibles dans le fluide, s'ils sont capturés par les éléments de capture (64) agencés au niveau des premières régions (66a).

10. Appareil de détection d'élément cible pour détecter des éléments cibles dans un fluide (3), dans lequel l'appareil de détection d'élément cible (38) est adapté pour pouvoir coopérer avec un dispositif capteur (1), dans lequel le dispositif capteur (1) comprend :
- un substrat (61) ayant des premières régions (66a) et des secondes régions (66b),
- une première couche à motifs (62) sur le substrat (61) couvrant les secondes régions (66b) de sorte que les premières régions (66a) ne soient pas couvertes par la première couche (62), dans lequel la première couche (62) est une couche de métal, et
- des éléments de capture (64) agencés dans les premières régions (66a) pour capturer les éléments cibles,
dans lequel l'appareil de détection d'élément cible (38) est adapté pour détecter les éléments cibles dans le fluide, s'ils sont capturés par les éléments de capture (64) agencés dans les premières régions (66a).

11. Procédé de fabrication d'un dispositif capteur qui est utilisable pour détecter des éléments cibles dans un fluide, comprenant les étapes de :
- fourniture d'un substrat (61) ;
- fourniture d'une première couche (62) sur le substrat (61), dans lequel la première couche (62) est une couche de métal ;
- mise en motifs de la première couche (62) obtenant des premières régions (66a) et des secondes régions (66b), dans lequel les secondes régions (66b) sont couvertes par la première couche à motifs (62) et les premières régions (66a) ne sont pas couvertes par la première couche à motifs (62) ; et
- la fourniture d'éléments de capture (64) pour capturer les éléments cibles du fluide dans les premières régions (66a).
